# EUROPÄISCHE PATENTSCHRIFT

(11) **EP 3 694 982 B1**
(45) Veröffentlichungstag und Bekanntmachung des Hinweises auf die Patenterteilung: **09.08.2023**
(21) Anmeldenummer: 18785906.1
(22) Anmeldetag: 08.10.2018
(51) Int. Cl.: C12M 1/00, C12M 1/09, C12M 1/06, C12N 1/12, C12N 1/20

(54) **VERFAHREN UND SYSTEM ZUR HETEROTROPHEN UND MIXOTROPHEN KULTIVIERUNG VON MIKROALGEN**
METHOD AND SYSTEM FOR HETEROTROPHIC AND MIXOTROPHIC CULTIVATION OF MICROALGAE
PROCÉDÉ ET SYSTÈME POUR LA CULTURE HÉTÉROTROPHE ET MIXOTROPHE DE MICROALGUES

(30) Priorität: 10.10.2017 DE 102017218001
(43) Veröffentlichungstag der Anmeldung: 19.08.2020
(73) Patentinhaber: Gicon Grossmann Ingenieur Consult GmbH, 01219 Dresden (DE)
(72) Erfinder: GROSSMANN, Jochen, 01187 Dresden (DE); COTTA, Fritz, 06217 Merseburg (DE); ECKE, Martin, 38486 Klötze (DE)
(74) Vertreter: Kailuweit & Uhlemann Patentanwälte Partnerschaft mbB
(86) Internationale Anmeldenummer: PCT/EP2018/077264
(87) Internationale Veröffentlichungsnummer: WO 2019/072738

(56) Entgegenhaltungen:
- EP-A1- 2 316 917
- WO-A1-2014/074772
- WO-A1-2014/144270
- DE-A1-102008 059 562
- US-A1- 2017 218 319
- OGBONNA JAMES C ET AL: "Light requirement and photosynthetic cell cultivation: Development of processes for efficient light utilization in photobioreactors", 1. Oktober 2000 (2000-10-01), JOURNAL OF APPLIED PHYCOLOGY, KLUWER, DORDRECHT, NL, PAGE(S) 207 - 218, XP002473562, ISSN: 0921-8971 in der Anmeldung erwähnt Seite 212 - Seite 215; Abbildung 5
- JAMES C OGBONNA ET AL: "Sequential heterotrophic/autotrophic cultivation - An efficient method of producing Chlorella biomass for health food and animal feed", 1. Juli 1997 (1997-07-01), JOURNAL OF APPLIED PHYCOLOGY, KLUWER ACADEMIC PUBLISHERS, DO, PAGE(S) 359 - 366, XP019247857, ISSN: 1573-5176 das ganze Dokument

## Beschreibung

Die Erfindung betrifft ein Verfahren zur simultanen heterotrophen und mixotrophen Kultivierung von Mikroalgen, ein System zur simultanen heterotrophen und mixotrophen Kultivierung von Mikroalgen sowie die Verwendung des Systems zur Kultivierung von Mikroalgen.

Mikroalgen sind eine wertvolle Ressource für die Produktion von Lebens- und Futtermitteln, Feinchemikalien, Arzneimitteln sowie kosmetischen Erzeugnissen, da sie in der Lage sind aus dem in der Luft enthaltenem Kohlenstoffdioxid und essentiellen mineralischen Nährstoffen in Gegenwart von Licht unter Freisetzung von Sauerstoff organische Substanzen aufzubauen. Dieser Vorgang wird als Phototrophie bezeichnet.

Eine beträchtliche Anzahl Mikroalgen ist weiterhin in der Lage, aus geeigneten organischen Substraten und essentiellen mineralischen Nährstoffen in Gegenwart von Sauerstoff auch in Abwesenheit von Licht unter Ausnutzug der in den organischen Substraten gespeicherten chemischen Energie organische Biomasse aufzubauen. Dieser Vorgang wird als Heterotrophie bezeichnet. Wiederum ein Teil dieser Mikroalgen kann simultan phototroph und heterotroph agieren. Diese Form wird als Mixotrophie bezeichnet.

Eine Voraussetzung für die phototrophe Kultivierung im industriell relevanten Maßstab ist die ausreichende Verfügbarkeit von anorganischem Kohlenstoff im Kultivierungssystem. Der Zusatz von anorganischem Kohlenstoff erfolgt hauptsächlich durch Eintrag von gasförmigem Kohlenstoffdioxid oder Hydrogenkarbonat in das Kulturmedium. Eine weitere Voraussetzung für die phototrophe Kultivierung ist die Bereitstellung der erforderlichen Lichtintensitäten in der Kultursuspension durch konstruktive Maßnahmen, insbesondere durch eine Führung des Kulturmediums in möglichst dünnen Schichten, sowie zur Sicherung der erforderlichen Nährstoffversorgung, des Gasaustausches und der Lichtverfügbarkeit in dichten Kulturen möglichst hohe Turbulenzen innerhalb des Kulturmediums.

Für die phototrophe Kultivierung von Mikroalgen im industriellen Maßstab haben sich zwei verschiedene Formen von Kultivierungssystemen durchgesetzt (Lee 2001). Eine Form ist die Kultivierung von Mikroalgen in offenen Vorrichtungen. Als offene Vorrichtungen oder offene Systeme werden Anlagen bezeichnet, in denen die zu kultivierenden Mikroalgen in direktem Kontakt mit der Umgebung stehen, um den für den Kultivierungsprozess erforderlichen Lichteintrag zu gewährleisten. Offene Vorrichtungen zur Mikroalgenkultivierung können natürliche Seen, Teiche, Becken ("Ponds") und künstliche Flussläufe ("Raceway Ponds") darstellen, in denen unter Zufuhr von Kohlenstoffdioxid aus der Luft bzw. durch direkte Einspeisung von Kohlenstoffdioxid oder Kohlenstoffdioxid-Gemischen in das wässrige, die erforderlichen mineralischen Nährstoffe enthaltende Kulturmedium die Mikroalgen kultiviert werden. Alternativ kann innerhalb entsprechender pH-Bereiche bei bestimmten Algenarten auch Hydrogencarbonat als Kohlenstoffquelle fungieren. Der erforderliche Gasaustausch einschließlich des Austrags des bei der Photosynthese gebildeten Sauerstoffs erfolgt an der Gewässeroberfläche in direktem Kontakt mit der Umgebungsluft. Die Durchmischung des Mediums kann durch Wind, Einblasen von Luft, durch Rührwerke oder auch Umpumpen des Mediums erfolgen. Um den erforderlichen Lichteintrag, insbesondere durch Sonnenlicht, sicherzustellen, ist die Gewässertiefe in den meisten Fällen kleiner als 30 cm. Offene Systeme können zusätzlich mit transparenten Abdeckungen versehen werden um Witterungseinflüsse und Schadstoffeinträge zu verringern.

Nachteile der offenen Systeme sind eine hohe Anfälligkeit gegenüber Kontaminationen mikrobieller und chemischer Natur, hohe Wasserverluste durch Verdunstung, eine geringe Photosyntheseeffizienz aufgrund des niedrigen atmosphärischen Kohlenstoffdioxid-partialdrucks sowie niedrige Biomassedichten, insbesondere kleiner 1 g/l, sowie geringe Raum-Zeitausbeuten.

In ausgewählten Fällen können offene Systeme auch mixotroph betrieben werden. Derartige Verfahren sind auf wenige, sehr schnell wachsende und eine hypertrophe Umgebung bevorzugende Algenarten beschränkt, z. B. Vertreter aus dem Chlorella-Cluster, die auch die für diese Prozesse immanenten Begleitbakterien tolerieren. Als organische Kohlenstoffquelle fungiert bei derartigen Verfahren bevorzugt Acetat. Die Anwendungsbreite mixotropher Verfahren auf offene Systeme ist auf Grund des durch den Zusatz organischer Nährstoffe drastisch erhöhten mikrobiellen Kontaminationsrisikos gering. Produkte aus derartigen Verfahren müssen aufwändig gereinigt und pasteurisiert werden.

Eine weitere Form der phototrophen Kultivierung von Mikroalgen ist die Kultivierung in geschlossenen Vorrichtungen, auch Photobioreaktoren (PBR) genannt. Als Photobioreaktor werden Vorrichtungen bezeichnet, in denen sich, von der Umgebung abgeschirmt, in transparenten Reaktionsräumen das die Mikroalgen enthaltene Kulturmedium befindet. Bevorzugt werden röhren- oder quaderförmige Vorrichtungen sowie auf Schlauchbeuteln beruhende Systeme genutzt. Als bevorzugte Materialien für die Vorrichtungen kommen Glas sowie diverse lichtdurchlässige Polymere, wie Polyvinylchlorid (PVC), Polyacrylat, Polyethylen oder Silikon (DE 102009045851 A1) zum Einsatz. In geschlossenen Vorrichtungen wird das für die Photosynthese erforderliche Kohlenstoffdioxid eingespeist sowie der bei der Photosynthese freigesetzte Sauerstoff ausgetragen, wobei höhere Kohlenstoffdioxid-partialdrücke und somit höhere Biomassedichten und eine höhere Produktivität als in offen Systemen erreicht werden. Zur Optimierung der Lichtausbeute und Erhöhung der Raum- Zeitausbeuten wird die Schichtdicke des Kulturmediums geringgehalten und die Erzeugung der erforderlichen Turbulenzen erfolgt durch Umpumpen der Suspension oder Einleiten von Prozessgasen. Vorteilhaft wird mittels geschlossener Systeme im Vergleich zu offenen Systemen das Kontaminationsrisiko durch Xenobiotika gesenkt, wobei das Risiko mikrobiologischer Kontaminationen aufgrund der in großtechnischen Anlagen üblichen unsterilen Fahrweise nur unwesentlich geringer ist.

Geschlossene Systeme werden weiterhin für die mixotrophe oder heterotrophe Kultivierung genutzt, wobei zur heterotrophen Kultivierung Tanks oder herkömmliche Fermenter-Systeme genutzt werden. Eine Voraussetzung für eine großvolumige heterotrophe Kultivierung ist eine sterile Prozessführung. Vorteilhaft werden bei der heterotrophen Kultivierung unter Zufuhr von Sauerstoff und einer Kohlenstoffquelle, insbesondere einer organischen Kohlenstoffquelle, bevorzugt Glucose, höhere Biomassedichten in der Kultursuspension erhalten. Nachteilig unterscheidet sich die Zusammensetzung heterotroph erzeugter Mikroalgenbiomasse von phototroph erzeugter Mikroalgenbiomasse, wobei einige wirtschaftlich interessante Inhaltsstoffe, z. B. Vitamine und Carotinoide, nur unter phototropher Kultivierung gebildet werden.

Eine mögliche Alternative bietet die Kombination von heterotropher und phototropher Kultivierung von zur dualen Trophie befähigter Mikroalgen. DE 10 2008 059 562 A1 offenbart ein Kultivierungsverfahren einer heterotrophen Mikroalge umfassend die heterotrophe Kultivierung und die Kultivierung in einem Photobioreaktor unter autotrophen oder mixotrophen Bedingungen, bevorzugt unter autotrophen Bedingungen.

Ogbonna und Tanaka beschreiben den Lichtbedarf und die effiziente Lichtausnutzung in Photobioreaktoren, insbesondere durch eine Kombination von heterotropher und phototropher Kultivierung oder eine sequentielle oder zyklische heterotrophe/phototrophe Kultivierung (Ogbonna und Tanaka 2000). Das Dokument offenbart neben separat betriebenen photoautotrophen und photoheterotrophen, also mixotrophen Kultivierungsprozessen eine sequentielle heterotroph / photoautotrophe Kultivierung, bei der nach stattgefundener heterotropher Kultivierung der Prozess in einer separaten Kultivierungseinheit unter photoautotrophen Bedingungen bis zur Entstehung der gewünschten Produkte weitergeführt wird. Weiterhin wird eine, als zyklische Prozessführung bezeichnete Variante zur Mikroalgenkultivierung offenbart, die auf periodisch aufeinander folgenden heterotrophen und photoautotrophen Verfahrensschritten beruht.

Weiterhin offenbart Ogbonna *et al.* die sequentielle heterotrophe/phototrophe Kultivierung von *Chlorella* mittels Fermenter und tubulärem Photobioreaktor bzw. von innen beleuchtetem Photobioreaktor, wobei eine Erhöhung des Protein- sowie Chlorophyllgehalts erreicht wird (Ogbonna *et al.* 1997). Der Übergang aus der heterotrophen in die phototrophe Kultivierung erfolgt dabei nach dem vollständigen Verbrauch der organischen Kohlenstoffquelle, um mikrobiologische Kontaminationen zu unterdrücken.

Bei der Kombination von heterotropher und phototropher Kultivierung in einer Vorrichtung unter Lichteinwirkung und in Gegenwart einer organischen Kohlenstoffquelle kommt es zur mixotrophen Assimilation, wobei vorteilhaft sonst nur phototroph zugängliche Inhaltsstoffe gebildet werden, ohne dass unproduktive Adaptionszeiten bei dem Übergang in die jeweils andere Kultivierungsform auftreten. Eine Voraussetzung für die mixotrophe Assimilation ist eine ausreichende Lichtversorgung bei den für mixotrophe Prozesse erforderlichen Schwachlichtbedingungen, weshalb nur mit geringen Biomassedichten gearbeitet werden kann.

Daher besteht die Aufgabe der vorliegenden Erfindung darin, ein Verfahren zur heterotrophen und mixotrophen Kultivierung von Mikroalgen unter Vermeidung der voranstehend geschilderten Nachteile miteinander zu kombinieren.

Ferner ist es Aufgabe der Erfindung, ein Verfahren zur Kultivierung von Mikroalgen mit hohen Biomassedichten und hohen Raum-Zeitausbeuten bereitzustellen.

Erfindungsgemäß wird die Aufgabe gelöst durch das erfindungsgemäße Verfahren zur simultanen heterotrophen und mixotrophen Kultivierung von Mikroalgen umfassend die Schritte
a) Bereitstellen eines Inokulums umfassend mindestens einen Mikroalgenstamm und Inokulation eines Kulturmediums mit dem Inokulum,
b) Kultivierung des mindestens einen Mikroalgenstamms in einer ersten Vorrichtung unter heterotrophen Kulturbedingungen,
c) Kultivierung des mindestens einen Mikroalgenstamms in einer zweiten Vorrichtung unter mixotrophen Kulturbedingungen,

wobei eine Förderung zumindest einer Teilmenge des mindestens einen Mikroalgenstamms aus der ersten Vorrichtung in Schritt b) in die zweite Vorrichtung in Schritt c) und/oder aus der zweiten Vorrichtung in Schritt c) in die erste Vorrichtung in Schritt b)erfolgt,
wobei ein Prozessgas an dem unteren Ende der zweiten Vorrichtung eingeleitet wird,
wobei das Prozessgas zumindest teilweise verdichtet oder zumindest teilweise kondensiert eingeleitet wird.

Vorteilhaft ermöglicht das erfindungsgemäße Verfahren zur Kultivierung von Mikroalgen hohe Biomassedichten und hohe Raum-Zeitausbeuten. Weiterhin vorteilhaft bilden die Mikroalgen, kultiviert durch das erfindungsgemäße Verfahren, Inhaltsstoffe, welche nur bei einer phototrophen Kultivierung gebildet werden. Weiterhin vorteilhaft können die mittels des erfindungsgemäßen Verfahrens kultivierten Mikroalgen ohne weitere Adaptionsphasen für nachfolgende, insbesondere heterotrophe, phototrophe oder mixotrophe Verfahrensschritte; eingesetzt werden oder einer direkten Weiterverarbeitung unterzogen werden.

Erfindungsgemäß ist eine "simultane heterotrophe und mixotrophe Kultivierung von Mikroalgen" die gleichzeitige heterotrophe Kultivierung nach Schritt b) und mixotrophe Kultivierung nach Schritt c) zumindest einer Teilmenge des mindestens einen Mikroalgenstamms. Bevorzugt erfolgt die simultane heterotrophe und mixotrophe Kultivierung von Mikroalgen in separaten Bereichen eines Reaktorsystems, insbesondere in einer ersten und einer zweiten Vorrichtung, welche dauerhaft durch mindestens ein Verbindungselement miteinander verbunden sind.

Erfindungsgemäß wird unter "heterotropher Kultivierung" eine Kultivierung in Gegenwart von organischen Substraten, essentiellen mineralischen Nährstoffen und Sauerstoff verstanden. Vorteilhaft ermöglicht die heterotrophe Kultivierung die Bereitstellung von hohen Konzentrationen an Mikroalgen.

Unter "organischen Substraten" werden organische Kohlenstoffquellen verstanden. In einer Ausführungsform sind organische Substrate ausgewählt aus Glucose, Essigsäure, Acetaten, Lactaten, Ethanol, Harnstoff und/oder Glutamat. In einer weiteren Ausführungsform sind organische Substrate ausgewählt aus filtrierten und sterilisierten, flüssigen Gärresten aus der Biogasgewinnung, Hydrolysaten aus stärkehaltigen Rohstoffen, bevorzugt Kartoffel- oder Maisschlempe oder Hefeextrakten.

Unter "essentiellen mineralischen Nährstoffen" werden Stickstoffverbindungen, insbesondere Nitrate, Nitrite, Ammoniak, Ammoniumverbindungen und/oder Harnstoff; Phosphorverbindungen, insbesondere Phosphate und/oder Phosphite; Schwefelverbindungen, insbesondere Sulfate; und Kalium-, Magnesium-, Calcium-, Eisenverbindungen und Verbindungen von Spurenelementen, insbesondere Borsäure, Cobaltsulfat, Kupfersulfat und/oder Zinksulfat, verstanden. In einer Ausführungsform umfassen essentielle mineralische Nährstoffe *in situ* mineralisierbare organische Phosphor- und Schwefelverbindungen, bevorzugt Phosphonate und/oder Methylsulphonylmethan.

Erfindungsgemäß wird unter "mixotropher Kultivierung" eine simultane phototrophe und heterotrophe Kultivierung verstanden.

Unter einer "phototrophen Kultivierung" wird eine Kultivierung in Gegenwart von essentiellen mineralischen Nährstoffen, Kohlenstoffdioxid und Licht verstanden.

Unter "Mikroalgen" werden mikroskopisch kleine, eukaryotische Lebewesen verstanden, welche im Wasser leben und Photosynthese durchführen. Unter mikroskopisch kleinen Lebewesen werden Lebewesen mit einer Länge von maximal 1 mm verstanden.

Erfindungsgemäß wird die Fähigkeit verschiedener Mikroalgen zu einem simultan phototroph und heterotroph generierten Wachstum ausgenutzt. In einer Ausführungsform des Verfahrens erfolgt die Kultivierung von heterotrophen Mikroalgen, bevorzugt Chlorophyten, *insbesondere Chlorella, Scenedesmus, Muriellopsis, Tetraselmis* besonders bevorzugt *Chlorella vulgaris, Chlorella sorokiniana, Chlorella regularis, Chlorella zofingiensis, Chlorella protothecoides, Parachlorella kessleri* oder *Scenedsmus vakuolatus;* oder Diatomeen, insbesondere *Odontella aurita* oder *Phaeodactylum trikornutum;* oder Euglena-Arten, insbesondere *Euglena gracilis;* oder Haptophyta, bevorzugt *Isochrysis species* oder *Pavlova species.* Unter einer "heterotrophen Mikroalge" wird eine Mikroalge verstanden, welche in Gegenwart von organischen Substraten, essentiellen mineralischen Nährstoffen und Sauerstoff auch in Abwesenheit von Licht organische Biomasse aufbaut.

Unter "Inokulum" werden Mikroalgenzellen zum Animpfen eines Kulturmediums in einer Vorrichtung verstanden, bevorzugt ein Mikroalgenzellen-enthaltendes erstes Kulturmedium zum Animpfen eines zweiten Kulturmediums in einer Vorrichtung. Unter "Inokulation" oder "Animpfen" wird das Hinzufügen eines replikationsfähigen Objekts, insbesondere einer Zelle, zu einem Kulturmedium verstanden. Bevorzugt wird unter "Inokulation" oder "Animpfen" das Hinzufügen eines ersten Kulturmediums enthaltend eine Zelle zu einem zweiten Kulturmedium verstanden.

Unter einem "Kulturmedium" wird eine wässrige Lösung zur Kultivierung von Mikroalgen verstanden. In einer Ausführungsform ist das Kulturmedium eine wässrige Pufferlösung. In einer bevorzugten Ausführungsform umfasst das Kulturmedium organische Substrate und/oder essentielle mineralische Nährstoffe.

In einer Ausführungsform des Verfahrens umfasst die Inokulation eines Kulturmediums mit dem Inokulum in Schritt a) einzelne Subkultivierungsschritte mit ansteigenden Kulturvolumina, bevorzugt drei Subkultivierungsschritte, wobei besonders bevorzugt jeweils eine Verdünnung mit Kulturmedium mit einem Verdünnungsfaktor von 10 erfolgt

In einer Ausführungsform des Verfahrens erfolgen die einzelnen Subkultivierungsschritte unter den gleichen Bedingungen, bevorzugt mit dem gleichen Kulturmedium, wie die Kultivierung des mindestens einen Mikroalgenstamms in einer ersten Vorrichtung unter heterotrophen Kulturbedingungen in Schritt b).

In einer Ausführungsform des Verfahrens wird das Kulturmedium nach der Inokulation in Schritt a) in die erste Vorrichtung zur Kultivierung des mindestens einen Mikroalgenstamms unter heterotrophen Kulturbedingungen in Schritt b) überführt.

In einer Ausführungsform des Verfahrens erfolgt ein getakteter oder kontinuierlicher Zusatz eines organischen Substrats in der Kultivierung des mindestens einen Mikroalgenstamms in einer ersten Vorrichtung unter heterotrophen Kulturbedingungen in Schritt b).

In einer Ausführungsform des Verfahrens erfolgt die Kultivierung des mindestens einen Mikroalgenstamms in einer ersten Vorrichtung unter heterotrophen Kulturbedingungen in Schritt b) unter Rühren, bevorzugt mit einer Rührgeschwindigkeit von 10 U/min bis 1000 U/min.

Vorteilhaft führt die Einleitung des Prozessgases an dem unteren Ende der zweiten Vorrichtung sowie die zumindest teilweise Verdichtung oder zumindest teilweise Kondensation des Prozessgases zur Bildung von Gasblasen, welche die zweite Vorrichtung durchströmen, wodurch dünne Schichten in der Mikroalgenkultur zwischen Wandung der zweiten Vorrichtung und Gasblasen erzeugt werden. Weiterhin vorteilhaft gewährleisten die dünnen Schichten eine ausreichende Versorgung der Mikroalgen mit Licht.

In einer Ausführungsform des Verfahrens ist das Prozessgas in Schritt c) Luft oder ein Kohlenstoffdioxid-angereichertes Luftgemisch, bevorzugt ein Abgas aus einer heterotrophen Kultivierung aus der ersten Vorrichtung oder hergestellt aus technischen Gasen. In einer Ausführungsform wird die Zusammensetzung und Menge des eingeleiteten Prozessgases in Schritt c) dem Prozessverlauf angepasst.

Unter "Luft" wird ein Gasgemisch umfassend Stickstoff und Sauerstoff und in geringen Mengen, bevorzugt unter 0,1 Vol.-%, Kohlenstoffdioxid verstanden. Unter einem "Kohlenstoffdioxidangereicherten Luftgemisch" wird ein Gasgemisch umfassend Stickstoff, Sauerstoff und Kohlenstoffdioxid mit einem Kohlenstoffdioxid-Anteil von 0,1 Vol.-% bis 20 Vol.-% verstanden.

In einer Ausführungsform des Verfahrens wird ein weiteres Prozessgas in die erste Vorrichtung in Schritt b) eingeleitet, wobei das weitere Prozessgas bevorzugt ein Sauerstoff-haltiges Gas oder Gasgemisch ist. Unter einem "Sauerstoff-haltigen Gas oder Gasgemisch" wird ein Gas oder Gasgemisch enthaltend Sauerstoff mit einem Anteil von 21 Vol.-% bis 100 Vol.-% verstanden.

In einer bevorzugten Ausführungsform des Verfahrens wird die Zusammensetzung und Menge des eingeleiteten Sauerstoff-haltigen Gases oder Gasgemisches in Schritt b) dem Prozessverlauf angepasst.

In einer Ausführungsform des Verfahrens wird das Prozessgas getaktet oder kontinuierlich eingeleitet. Unter "getaktet" wird eine Einleitung des Prozessgases in Form von aufeinanderfolgenden, voneinander abgegrenzten Gasblasen verstanden. Bevorzugt wird das Prozessgas getaktet mit einer Injektionszeit von 0,1 s bis 10 s mit 0,5 bis 50 Injektionen pro Minute eingeleitet.

In einer Ausführungsform des Verfahrens beträgt die Menge des eingeleiteten Prozessgases 1 cm³/s bis 100.000 cm³/s, bevorzugt 1 cm³/s bis 10.000 cm³/s, besonders bevorzugt 10 cm³/s bis 1.000 cm³/s.

In einer Ausführungsform des Verfahrens ist der Druck des eingeleiteten Prozessgases 0,3 bar bis 5 bar über dem hydrostatischen Druck der ersten und zweiten Vorrichtung, bevorzugt 0,5 bis 2,5 bar über dem hydrostatischen Druck der ersten und zweiten Vorrichtung, besonders bevorzugt 0,5 bis 1,5 bar über dem hydrostatischen Druck der ersten und zweiten Vorrichtung.

In einer Ausführungsform des Verfahrens ist der Anlagendruck der ersten Vorrichtung und der zweiten Vorrichtung gleich.

In weiteren Ausführungsformen des Verfahrens erfolgt eine getaktete oder kontinuierliche Förderung zumindest einer Teilmenge des mindestens einen Mikroalgenstamms aus der ersten Vorrichtung in Schritt b) in die zweite Vorrichtung in Schritt c) und/oder aus der zweiten Vorrichtung in Schritt c) in die erste Vorrichtung in Schritt b).

In bevorzugten Ausführungsformen des Verfahrens erfolgt eine getaktete oder kontinuierliche Förderung zumindest einer Teilmenge des mindestens einen Mikroalgenstamms aus der ersten Vorrichtung in Schritt b) in die zweite Vorrichtung in Schritt c) und aus der zweiten Vorrichtung in Schritt c) in die erste Vorrichtung in Schritt b).

In einer besonders bevorzugten Ausführungsform liegt die Verweilzeit der Teilmenge des mindestens einen Mikroalgenstamms in der zweiten Vorrichtung zwischen 60 s und 3600 s.

Bevorzugt erfolgt die getaktete oder kontinuierliche Förderung durch die Einleitung eines Prozessgases in Schritt c) oder durch eine Pumpe oder durch die Einleitung eines Prozessgases in Schritt c) und eine Pumpe.

In einer Ausführungsform des Verfahrens beträgt das Volumen der getaktet oder kontinuierlich geförderten Teilmenge des mindestens einen Mikroalgenstamms aus der ersten Vorrichtung in Schritt b) in die zweite Vorrichtung in Schritt c) und/oder aus der zweiten Vorrichtung in Schritt c) in die erste Vorrichtung in Schritt b) das Doppelte bis das Zehnfache des Füllvolumens der zweiten Vorrichtung pro Stunde, bevorzugt das Vierfache des Füllvolumens der zweiten Vorrichtung pro Stunde. Unter Füllvolumen wird das Volumen des mindestens einen Mikroalgenstamms in Kulturmedium in einer Vorrichtung verstanden.

In einer Ausführungsform des Verfahrens ist die Temperatur des mindestens einen Mikroalgenstamms in Kulturmedium in der ersten Vorrichtung und/oder in der zweiten Vorrichtung im Bereich von 10 °C bis 40 °C. In einer weiteren Ausführungsform ist die Differenz der Temperaturen des mindestens einen Mikroalgenstamms in Kulturmedium in der ersten Vorrichtung und in der zweiten Vorrichtung 1 K bis 10 K.

In einer Ausführungsform des Verfahrens ist der pH-Wert des mindestens einen Mikroalgenstamms in Kulturmedium in der ersten Vorrichtung und/oder in der zweiten Vorrichtung im Bereich von pH 5.5 bis pH 9.5, bevorzugt im Bereich von pH 6.0 bis pH 8.5, besonders bevorzugt im Bereich von pH 6.5 bis pH 7.5.

In weiteren Ausführungsformen umfasst das erfindungsgemäße Verfahren mindestens einen weiteren Schritt, wobei der weitere Schritt ausgewählt ist aus einer phototrophen Kultivierung, einer mixotrophen Kultivierung, einer Ernte der Biomasse und/oder dem Trocknen der geernteten Biomasse. Unter "Ernte der Biomasse" wird das Abtrennen der Mikroalgen von dem Kulturmedium verstanden.

Gegenstand der Erfindung ist auch ein System zur simultanen heterotrophen und mixotrophen Kultivierung von Mikroalgen umfassend
i. eine erste Vorrichtung, wobei die erste Vorrichtung zur heterotrophen Kultivierung ausgebildet ist,
ii. eine zweite Vorrichtung umfassend ein Rohr aus transluzentem Material und mindestens eine Lichtquelle, wobei die zweite Vorrichtung zur mixotrophen Kultivierung ausgebildet ist,
iii. ein erstes Verbindungselement zur Verbindung der ersten Vorrichtung mit der zweiten Vorrichtung,
   wobei das erste Verbindungselement den Boden der ersten Vorrichtung mit dem Einlass der zweiten Vorrichtung verbindet,
iv. ein zweites Verbindungselement zur Verbindung der ersten Vorrichtung mit der zweiten Vorrichtung,
   wobei das zweite Verbindungselement den oberen Teil der ersten Vorrichtung mit dem Auslass der zweiten Vorrichtung verbindet,
   wobei die zweite Vorrichtung an dem unteren Ende oder das erste Verbindungselement einen Injektor zur Einleitung eines Prozessgases umfasst.

Unter dem Einlass der zweiten Vorrichtung wird das untere Ende der zweiten Vorrichtung verstanden und unter dem Auslass der zweiten Vorrichtung wird das obere Ende der zweiten Vorrichtung verstanden.

In einer Ausführungsform ist das erfindungsgemäße System ein geschlossenes System. Vorteilhaft verringert ein geschlossenes System das Risiko für Kontaminationen. Weiterhin vorteilhaft wird ein höherer Prozessgasdruck erreicht. Weiter vorteilhaft wird die Verdunstung des Kulturmediums verhindert. Weiterhin vorteilhaft werden höhere Biomassedichten in der Kultursuspension erreicht.

Bevorzugt ist die erste Vorrichtung, ausgebildet zur heterotrophen Kultivierung, ein steril betreibbarer, geschlossener Rührkessel, besonders bevorzugt mit einem Verhältnis von Durchmesser zu Höhe im Bereich von 1:1 bis 1:3.

In einer Ausführungsform ist die erste Vorrichtung ein Fermenter, bevorzugt ein Temperaturregelbarer, pH-Wert-regelbarer und/oder Prozessgaszufuhr-regelbarer Fermenter. In einer Ausführungsform umfasst der Fermenter eine Rühreinrichtung, bevorzugt ein Rührwerk, besonders bevorzugt ein Rushton-Turbinen-Rührwerk; und/oder einen Injektor zur Einleitung eines Prozessgases.

Bevorzugt ist das Rohr aus transluzentem Material ein Strömungsrohr. Unter einem Strömungsrohr wird ein Rohr verstanden, welches eine getaktete oder kontinuierliche Förderung von Mikroalgen ermöglicht, insbesondere eine getaktete oder kontinuierliche Förderung einer Teilmenge mindestens einen Mikroalgenstamms mit einem Volumen im Bereich des Doppelten bis Zehnfachen des Füllvolumens des Rohrs aus transluzentem Material pro Stunde, bevorzugt des Vierfachen des Füllvolumens des Rohrs aus transluzentem Material pro Stunde.

In einer Ausführungsform ist das Rohr aus transluzentem Material eine Kollektoreinheit eines Photobioreaktors. Unter einer Kollektoreinheit eines Photobioreaktors wird der Teil eines Photobioreaktors verstanden, welcher Lichteintrittsflächen aufweist.

Bevorzugt weist das System zur simultanen heterotrophen und mixotrophen Kultivierung von Mikroalgen ein Verhältnis des Volumens der ersten Vorrichtung zu dem Volumen des Rohrs aus transluzentem Material im Bereich von 10:1 bis 1:5 auf.

In einer Ausführungsform des Systems ist das aus transluzentem Material bestehende Rohr spiralförmig aufsteigend um ein Trägergestell in Form eines Kegelstumpfs oder in Form eines Hohlzylinders angeordnet, wobei das Trägergestell mindestens eine Lichtquelle umfasst.

In einer Ausführungsform des Systems umfasst die zweite Vorrichtung mindestens eine weitere Lichtquelle zur Bestrahlung des Rohrs aus transluzentem Material von außen, wobei die mindestens eine weitere Lichtquelle bevorzugt ein Flächenstrahler ist.

In einer Ausführungsform des Systems ist das transluzente Material ausgewählt aus Glas oder lichtdurchlässigen Polymeren, bevorzugt Silikonen, Polyvinylchlorid (PVC), Polyacrylaten, Polyethylen oder Borosilikatglas.

In einer Ausführungsform des Systems umfasst das Rohr aus transluzentem Material mindestens ein weiteres Material ausgewählt aus UVB- und UVC-durchlässigen Materialien, bevorzugt Quarzglas.

In einer Ausführungsform des Systems ist die mindestens eine Lichtquelle ausgewählt aus einer Natriumdampflampe oder einem Leuchtdioden (LED)-Strahler.

In einer bevorzugten Ausführungsform des Systems weist die mindestens eine Lichtquelle eine Wellenlänge im Bereich von 400 nm bis 520 nm oder von 600 nm bis 720 nm auf.

In einer Ausführungsform des Systems weist die mindestens eine Lichtquelle eine Lichtleistung im Bereich von 1 W/m² bis 150 W/m² auf.

In einer Ausführungsform des Systems umfasst die zweite Vorrichtung mindestens eine weitere Lichtquelle, wobei die mindestens eine weitere Lichtquelle eine Wellenlänge im Bereich von 280 nm bis 320 nm aufweist.

In einer Ausführungsform des Systems ist die erste Vorrichtung und/oder die zweite Vorrichtung sterilisierbar. In einer Ausführungsform des Systems ist das erste Verbindungselement und/oder das zweite Verbindungselement sterilisierbar, bevorzugt ein sterilisierbarer Flansch.

In einer Ausführungsform des Systems umfasst die zweite Vorrichtung eine Pumpe, bevorzugt eine Drehkolbenpumpe. Bevorzugt ist die Pumpe nach dem ersten Verbindungselement angeordnet.

Ein weiterer Aspekt der Erfindung betrifft die Verwendung des erfindungsgemäßen Verfahrens oder des erfindungsgemäßen Systems zur Kultivierung von Mikroalgen.

Für die Realisierung der Erfindung ist es auch zweckmäßig, die vorbeschriebenen Ausführungsformen und Merkmale der Ansprüche zu kombinieren.

### Ausführungsbeispiele

Nachfolgend soll die Erfindung anhand einiger Ausführungsbeispiele und zugehöriger Figuren eingehender erläutert werden. Die Ausführungsbeispiele sollen dabei die Erfindung beschreiben ohne diese zu beschränken.

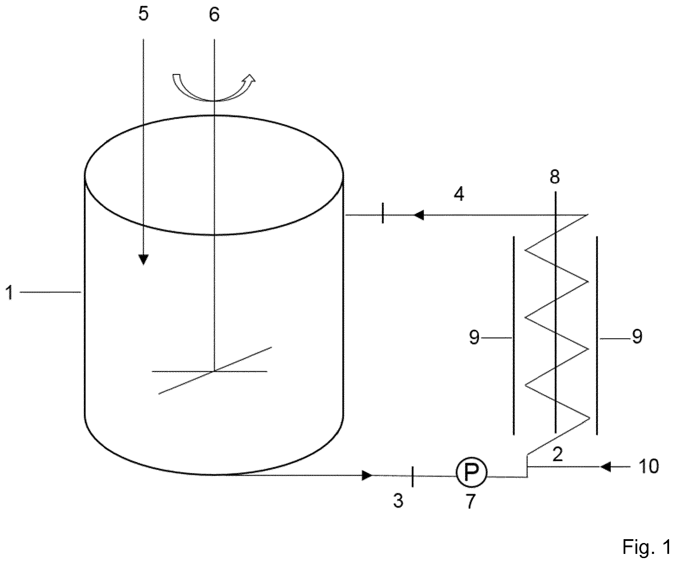

Fig. 1 zeigt ein Schema des erfindungsgemäßen Systems zur simultanen heterotrophen und mixotrophen Kultivierung von Mikroalgen umfassend eine erste Vorrichtung 1 und eine zweite Vorrichtung 2 umfassend ein Rohr aus transluzentem Material. Die erste Vorrichtung 1 ist zur heterotrophen Kultivierung ausgebildet und bevorzugt ein steril betreibbarer, geschlossener Rührkessel. Die erste Vorrichtung **1** umfasst bevorzugt einen Injektor zur Einleitung eines Prozessgases **5** und ein Rührwerk **6**, besonders bevorzugt ein Rushton-Turbinen-Rührwerk. Im Bodenbereich der ersten Vorrichtung **1** befindet sich ein erstes Verbindungselement **3** zur dichten Verbindung der ersten Vorrichtung **1** mit der zweiten Vorrichtung **2.** Die zweite Vorrichtung **1** umfasst bevorzugt eine Pumpe **7**, besonders bevorzugt eine sterilisierbare Drehkolbenpumpe. Die Pumpe **7** ist mit dem Rohr aus transluzentem Material verbunden, wobei sich zwischen Pumpe **7** und dem Rohr aus transluzentem Material ein Injektor zur Einleitung eines Prozessgases **10** für die mixotrophe Kultivierung befindet. Das Rohr aus transluzentem Material ist aufsteigend um ein Trägergestell (nicht gezeigt) angeordnet. Die erste Lichtquelle **8** ermöglicht eine Beleuchtung von innen und die zweite Lichtquelle **9** eine Beleuchtung von außen. Der Auslass bzw. das obere Ende des Rohrs aus transluzentem Material ist druckdicht mit dem oberen Teil der ersten Vorrichtung **1** über ein zweites Verbindungselement **4** verbunden.

### Ausführungsbeispiel 1:

Die Kultivierung von *Chlorella sorokiniana* erfolgt in einer erfindungsgemäßen Anlage entsprechend Fig. 1. Die erste Vorrichtung 1 und die zweite Vorrichtung 2 besitzen ein Arbeitsvolumen von jeweils 400 l.

Das System wird mit 600 l Kulturmedium l befüllt und sterilisiert. Das Kulturmedium l setzt sich wie folgt zusammen: 1,5 g/l KNOs; 50 mg/l KH₂PO₄; 100 mg/l MgSO₄-7H₂O; 14 mg/l FeSO₄-7H₂O; 1 mg/l MnSO₄-5H₂O; 0,7 mg ZnSO₄-7H₂O; 0,06 mg/l HsBOs; 0,024 mg/l CoSO₄-7H₂O; 0,024 mg/l CuSO₄·5H₂O; 0,01 mg/l (NH₄)₆Mo₇O₂₄·4H₂O sowie 10 g/l Glucose, wobei die Glucose separat sterilisiert und erst nach der Sterilisation des Mediums zugegeben wird. Das Kulturmedium wird über die erste Vorrichtung 1 mit 30 l ca. 20 g/l *Chlorella sorokiniana* enthaltendem Inokulum inokuliert und unter Rühren in der ersten Vorrichtung 1 kontinuierlich in die zweite Vorrichtung 2 und von dort zurück in die erste Vorrichtung 1 gepumpt. Die zweite Vorrichtung 2 wird mit Licht im PAR (photosynthetisch aktive Strahlung, *Photosynthetically Active* Radiation)-Bereich mit einer Intensität von 80 W/m², bezogen auf die als Kegelstumpf betrachtete Innenoberfläche der zweiten Vorrichtung, beleuchtet. Beide Vorrichtungen werden auf 32 °C temperiert und der pH-Wert mit Schwefelsäure (H₂SO₄) automatisch auf pH 6.8 eingestellt. Die *Chlorella sorokiniana-*Mikroalgen werden 4 Tage kultiviert, die Zufuhr der benötigten Glucose und Mineralstoffe erfolgt diskontinuierlich entsprechend des durch das Algenwachstum generierten Verbrauchs. Menge und Zusammensetzung der zugeführten Prozessgase, insbesondere des Kohlenstoff-dioxidangereicherten Gasgemisches und des Sauerstoff-haltigen Gasgemisches, werden entsprechend des Algenwachstums getrennt in der ersten Vorrichtung 1 und der zweiten Vorrichtung 2 geregelt. Nach 4 Tagen hat die Biomasse in der Anlage eine Konzentration von 35 g/l erreicht. Die Biomasse wird geerntet, mittels einer Zentrifuge auf 100 g/l angereichert und sprühgetrocknet. Die Biomasse hat einen Chlorophyllgehalt von 3,0 % und enthält 3500 ppm Lutein bezogen auf die Trockenmasse.

### Ausführungsbeispiel 2:

Die Kultivierung von *Chlorella sorokiniana* erfolgt in einer erfindungsgemäßen Anlage entsprechend Fig. 1. Die erste Vorrichtung 1 besitzt ein Arbeitsvolumen von 100 l und die zweite Vorrichtung 2 ein Arbeitsvolumen von 400 l.

Das System wird sterilisiert und mit 450 I Kulturmedium II befüllt. Das Kulturmedium II setzt sich wie folgt zusammen: 0,5 g/l KNOs; 1 g/l Harnstoff; 100 mg/l KH₂PO₃; 100 mg/l M₉SO₄·7H₂O; 14 mg/l FeSO₄-7H₂O; 1 mg/l MnSO₄·5H₂O; 0,7 mg ZnSO₄-7H₂O; 0,06 mg/l HsBOs; 0,024 mg/l C_{O}SO₄-7H₂O; 0,024 mg/l CuSO₄·5H₂O; 0,01 mg/l (NH₄)₆Mo₇O₂₄·4H₂O sowie 0,5 g/l Natriumacetat.

Das Kulturmedium wird über die erste Vorrichtung 1 mit 5 I ca. 100 g/l *Chlorella sorokiniana* enthaltendem Inokulum inokuliert. Das Inokulum wird vor der Inokulation mit 5 g Natriumacetat versetzt, der pH-Wert mit H₂SO₄ über einen Zeitraum von 30 Minuten auf pH 3.2 abgesenkt und anschließend mit einer Natriumhydroxid (NaOH)-Lösung auf pH 7.0 angehoben. Unter Rühren wird der Mikroalgenstamm und das Kulturmedium aus der ersten Vorrichtung 1 kontinuierlich in die zweite Vorrichtung 2 und von dort zurück in die erste Vorrichtung 1 gepumpt. Die zweite Vorrichtung 2 wird mit Licht im PAR-Bereich mit einer Intensität von 70 W/m², bezogen auf die als Kegelstumpf betrachtete Innenoberfläche der zweiten Vorrichtung, beleuchtet Beide Vorrichtungen werden auf 30 °C temperiert und der pH-Wert mit Essigsäure automatisch auf pH 7.0 eingestellt. Die Zufuhr der für die Kohlenstoffversorgung benötigten Essigsäure erfolgt pH-Wert geregelt diskontinuierlich entsprechend des durch das Algenwachstum generierten Verbrauchs. Weiterhin erfolgt mit der Zufuhr von Essigsäure auch die Zufuhr des erforderlichen Stickstoffs in Form von Ammoniumacetat sowie der Spurenelemente Eisen und Mangan. KH₂PO₄ wird separat entsprechend des durch das Algenwachstum generierten Verbrauchs dosiert. Menge und Zusammensetzung der zugeführten Prozessgase, insbesondere des Kohlenstoffdioxid-angereicherten Gasgemisches und des Sauerstoff-haltigen Gasgemisches, werden entsprechend des Algenwachstums getrennt in der ersten Vorrichtung 1 und der zweiten Vorrichtung 2 geregelt. Nach 8 Tagen hat die Biomasse in der Anlage eine Konzentration von 12 g/l erreicht. Die Biomasse wird geerntet, mittels einer Zentrifuge auf 100 g/l angereichert und sprühgetrocknet. Die Biomasse hat einen Chlorophyllgehalt von 3,5 % und enthält 4000 ppm Lutein bezogen auf die Trockenmasse.

### Ausführungsbeispiel 3:

Die Kultivierung von *Chlorella sorokiniana* erfolgt in einer erfindungsgemäßen Anlage entsprechend Fig. 1 als revolvierendes Verfahren. Die erste Vorrichtung 1 besitzt ein Arbeitsvolumen von 100 I und die zweite Vorrichtung 2 ein Arbeitsvolumen von 400 l.

Das System wird sterilisiert und mit 450 I Kulturmedium II befüllt. Das Kulturmedium II setzt sich wie folgt zusammen: 0,5 g/l KNOs; 1 g/l Harnstoff; 100 mg/l KH₂PO₃; 100 mg/l MgSO₄-7H₂O; 14 mg/l FeSO₄-7H₂O; 1 mg/l MnSO₄-5H₂O; 0,7 mg ZnSO₄-7H₂O; 0,06 mg/l HsBOs; 0,024 mg/l C_{O}SO₄-7H₂O; 0,024 mg/l CuSO₄·5H₂O; 0,01 mg/l (NH₄)₆Mo₇O₂₄·4H₂O sowie 0,5 g/l Natriumacetat.

Das Kulturmedium wird über die erste Vorrichtung 1 mit 5 I ca. 100 g/l *Chlorella sorokiniana* enthaltendem Inokulum inokuliert. Das Inokulum wird vor der Inokulation mit 5 g Natriumacetat versetzt, der pH-Wert mit H₂SO₄ über einen Zeitraum von 30 Minuten auf pH 3.2 abgesenkt und anschließend mit einer NaOH-Lösung auf pH 7.0 angehoben. Unter Rühren wird der Mikroalgenstamm und das Kulturmedium aus der ersten Vorrichtung 1 kontinuierlich in die zweite Vorrichtung 2 und von dort zurück in die erste Vorrichtung 1 gepumpt. Die zweite Vorrichtung 2 wird mit Licht im PAR-Bereich mit einer Intensität von 70 W/m², bezogen auf die als Kegelstumpf betrachtete Innenoberfläche der zweiten Vorrichtung, beleuchtet Beide Vorrichtungen werden auf 30 °C temperiert und der pH-Wert mit Essigsäure automatisch auf pH 7.0 eingestellt. Die Zufuhr der für die Kohlenstoffversorgung benötigten Essigsäure erfolgt pH-Wert geregelt diskontinuierlich entsprechend des durch das Algenwachstum generierten Verbrauchs. Weiterhin erfolgt mit der Zufuhr von Essigsäure auch die Zufuhr des erforderlichen Stickstoffs in Form von Ammoniumacetat sowie der Spurenelemente Eisen und Mangan. KH₂PO₄ wird separat entsprechend des durch das Algenwachstum generierten Verbrauchs dosiert. Menge und Zusammensetzung der zugeführten Prozessgase, insbesondere des Kohlenstoff-dioxidangereicherten Gasgemisches und des Sauerstoff-haltigen Gasgemisches, werden entsprechend des Algenwachstums getrennt in der ersten Vorrichtung 1 und der zweiten Vorrichtung 2 geregelt. Nach 8 Tagen hat die Biomasse in der Anlage eine Konzentration von 12 g/l erreicht. Die Biomasse wird geerntet, mittels einer Zentrifuge auf 100 g/l angereichert und sprühgetrocknet. Die Biomasse hat einen Chlorophyllgehalt von 3,5 % und enthält 4000 ppm Lutein bezogen auf die Trockenmasse. 5 I der geernteten und angereicherten Biomasse werden vor der Inokulation mit 5 g Natriumacetat versetzt, der pH-Wert mit H₂SO₄ über einen Zeitraum von 30 Minuten auf pH 3.2 abgesenkt und anschließend mit einer Natriumhydroxid (NaOH)-Lösung auf pH 7.0 angehoben. Mit dieser Biomasse wird das nach der Totalernte neu mit Kulturmedium II befüllte System ohne weitere Zwischendesinfektion inokuliert. Die Kultivierung wird mit den oben beschriebenen Verfahrensschritten nach der Inokulation fortgesetzt. Die geerntete Biomasse hat einen Chlorophyllgehalt von 3,5 % und enthält 4000 ppm Lutein bezogen auf die Trockenmasse.

### Ausführungsbeispiel 4:

12 I einer nach Ausführungsbeispiel 1 erhaltenen 10 %-igen *Chlorella* sorokiniana-Suspension werden zur Inokulation eines mit 1200 I Kulturmedium III befüllten tubulären Photobioreaktors benutzt. Kulturmedium III entspricht Kulturmedium I, enthält aber keine Glucose. In dem Photobioreaktor wird die Mikroalgenkultur über einen Zeitraum von 4 Wochen kultiviert. Die Kultivierung erfolgt im natürlichen Tagesrhythmus, durch zuschaltbare Zusatzbeleuchtung wird eine Mindestlichtintensität im PAR-Bereich von 80 W/m² in der Tagesphase, bezogen auf die als Kegelstumpf betrachtete Innenoberfläche der zweiten Vorrichtung, eingestellt. Der pH-Wert wird durch Einleitung von Kohlenstoffdioxid (CO₂) automatisch geregelt. Mineralische Nährstoffe und Spurenelemente werden entsprechend des durch das Algenwachstum generierten Verbrauchs dosiert. Nach 5 Tagen hat die Biomasse in der Anlage eine Konzentration von 3,5 g/l erreicht. Es werden 400 I Kultursuspension geerntet und durch frisches Kulturmedium III ersetzt. Dieser Prozess wird bis zum Ende der 4. Woche wiederholt, der letzte Erntezyklus wird als Totalernte gestaltet.

### Ausführungsbeispiel 5:

In einer alternativen Ausgestaltung von Ausführungsbeispiel 1 erfolgt eine Kultivierung von *Chlorella zofingiensis* in einer erfindungsgemäßen Anlage entsprechend Fig. 1, wobei eine Temperierung beider Vorrichtungen auf 28 °C erfolgt.

12 I der entsprechend erhaltenen 10%-igen *Chlorella* zofingiensis-Suspension werden zur Inokulation eines mit 1200 I Kulturmedium III befüllten tubulären Photobioreaktors benutzt. Kulturmedium III entspricht Kulturmedium I, enthält aber keine Glucose. In dem Photobioreaktor wird die Mikroalgenkultur über einen Zeitraum von 4 Wochen bei 28 °C Tagestemperatur und 20 °C Nachttemperatur kultiviert. Die Kultivierung erfolgt im natürlichen Tagesrhythmus, durch zuschaltbare Zusatzbeleuchtung wird eine Mindestlichtintensität im PAR-Bereich von 80 W/m² in der Tagesphase, bezogen auf die als Kegelstumpf betrachtete Innenoberfläche der zweiten Vorrichtung, eingestellt. Der pH-Wert wird durch Einleitung von Kohlenstoffdioxid (CO₂) automatisch geregelt. Mineralische Nährstoffe und Spurenelemente werden entsprechend des durch das Algenwachstum generierten Verbrauchs dosiert. Nach 5 Tagen hat die Biomasse in der Anlage eine Konzentration von 3,0 g/l erreicht. Es werden 400 I Kultursuspension geerntet und durch frisches Kulturmedium III ersetzt. Dieser Prozess wird bis zum Ende der 4. Woche wiederholt, der letzte Erntezyklus wird als Totalernte gestaltet. Die jeweils geerntete Biomasse enthält neben weiteren Carotinoiden 1300 ppm Astaxanthin, bezogen auf die Trockenmasse.

### Ausführungsbeispiel 6:

Die Kultivierung von *Chlorella vulgaris* erfolgt in einer erfindungsgemäßen Anlage entsprechend Fig. 1, wobei in der zweiten Vorrichtung 2 ein Bereich von 50 cm des Rohrs aus transluzentem Material durch ein separat beleuchtbares Rohr aus Quarzglas ersetzt wurde. Die erste Vorrichtung 1 besitzt ein Arbeitsvolumen von 100 I und die zweite Vorrichtung 2 ein Arbeitsvolumen von 400 l.

Das System wird sterilisiert und mit 450 I Kulturmedium II befüllt. Das Kulturmedium II setzt sich wie folgt zusammen: 0,5 g/l KNOs; 1 g/l Harnstoff; 100 mg/l KH₂PO₃; 100 mg/l MgSO₄·7H₂O; 14 mg/l FeSO₄·7H₂O; 1 mg/l MnSO₄·5H₂O; 0,7 mg ZnSO₄·7H₂O; 0,06 mg/l HsBOs; 0,024 mg/l C_{O}SO₄·7H₂O; 0,024 mg/l CuSO₄·5H₂O; 0,01 mg/l (NH₄)₆Mo₇O₂₄·4H₂O sowie 0,5 g/l Natriumacetat.

Das Kulturmedium wird über die erste Vorrichtung 1 mit 5 I ca. 100 g/l *Chlorella vulgaris* enthaltendem Inokulum inokuliert. Das Inokulum wird vor der Inokulation mit 5 g Natriumacetat versetzt, der pH-Wert mit H₂SO₄ über einen Zeitraum von 30 Minuten auf pH 3.2 abgesenkt und anschließend mit einer NaOH-Lösung auf pH 7.0 angehoben. Unter Rühren wird der Mikroalgenstamm und das Kulturmedium aus der ersten Vorrichtung 1 kontinuierlich in die zweite Vorrichtung 2 und von dort zurück in die erste Vorrichtung 1 gepumpt. Die zweite Vorrichtung 2 wird mit Licht im PAR-Bereich mit einer Intensität von 70 W/m², bezogen auf die als Kegelstumpf betrachtete Innenoberfläche der zweiten Vorrichtung, beleuchtet. Der aus Quarzglas bestehende Abschnitt des Rohres wird UVB-Strahlung ausgesetzt. Beide Vorrichtungen werden auf 27 °C temperiert und der pH-Wert mit Essigsäure automatisch auf pH 7.0 eingestellt. Die Zufuhr der für die Kohlenstoffversorgung benötigten Essigsäure erfolgt pH-Wert geregelt diskontinuierlich entsprechend des durch das Algenwachstum generierten Verbrauchs. Weiterhin erfolgt mit der Zufuhr von Essigsäure auch die Zufuhr des erforderlichen Stickstoffs in Form von Ammoniumacetat sowie der Spurenelemente Eisen und Mangan. KH₂PO₄ wird separat entsprechend des durch das Algenwachstum generierten Verbrauchs dosiert. Menge und Zusammensetzung der zugeführten Prozessgase, insbesondere des Kohlenstoff-dioxidangereicherten Gasgemisches und des Sauerstoff-haltigen Gasgemisches, werden entsprechend des Algenwachstums getrennt in der ersten Vorrichtung 1 und der zweiten Vorrichtung 2 geregelt. Nach 8 Tagen hat die Biomasse in der Anlage eine Konzentration von 8 g/l erreicht. Die Biomasse wird geerntet, mittels einer Zentrifuge auf 100 g/l angereichert und sprühgetrocknet. Die Biomasse hat einen Chlorophyllgehalt von 3,5 % und enthält neben Carotinoiden 0,05 ppm Vitamin D2, bezogen auf die Trockenmasse.

### Zitierte Nichtpatentliteratur

Lee Y-K (2001) Microalgal mass systems and methods: Their limitation and potential. Journal of Applied Phycology 13: 307-315.

Ogbonna JC, Tanaka H (2000) Light requirement and photosynthetic cell cultivation-Development of processes for efficient light utilization in photobioreactors. Journal of Applied Phycology 12: 207-218.

Ogbonna JC, Masui H, Tanaka H (1997) Sequential heterotrophic/autotrophic cultivation - An efficient method of producing Chlorella biomass for health food and animal feed. Journal of Applied Phycology 9: 359-366.

### Bezugszeichen

- 1: erste Vorrichtung, bevorzugt ein steril betreibbarer, geschlossener Rührkessel
- 2: zweite Vorrichtung umfassend ein Rohr aus transluzentem Material
- 3: erstes Verbindungselement
- 4: zweites Verbindungselement
- 5: Injektor zur Einleitung eines Prozessgases
- 6: Rührwerk
- 7: Pumpe
- 8: erste Lichtquelle
- 9: zweite Lichtquelle
- 10: Injektor zur Einleitung eines Prozessgases

## Patentansprüche

1. Verfahren zur simultanen heterotrophen und mixotrophen Kultivierung von Mikroalgen umfassend die Schritte
a) Bereitstellen eines Inokulums umfassend mindestens einen Mikroalgenstamm und Inokulation eines Kulturmediums mit dem Inokulum,
b) Kultivierung des mindestens einen Mikroalgenstamms in einer ersten Vorrichtung unter heterotrophen Kulturbedingungen,
c) Kultivierung des mindestens einen Mikroalgenstamms in einer zweiten Vorrichtung unter mixotrophen Kulturbedingungen,
wobei eine Förderung zumindest einer Teilmenge des mindestens einen Mikroalgenstamms aus der ersten Vorrichtung in Schritt b) in die zweite Vorrichtung in Schritt c) und/oder aus der zweiten Vorrichtung in Schritt c) in die erste Vorrichtung in Schritt b) erfolgt,
wobei ein Prozessgas an dem unteren Ende der zweiten Vorrichtung eingeleitet wird,
wobei das Prozessgas zumindest teilweise verdichtet oder zumindest teilweise kondensiert eingeleitet wird.

2. Verfahren nach Anspruch 1, **dadurch gekennzeichnet, dass** das Prozessgas in Schritt c) Luft oder ein Kohlenstoffdioxid-angereichertes Luftgemisch ist, bevorzugt ein Abgas aus einer heterotrophen Kultivierung aus der ersten Vorrichtung oder hergestellt aus technischen Gasen.

3. Verfahren nach Anspruch 1 oder 2, **dadurch gekennzeichnet, dass** das Prozessgas getaktet oder kontinuierlich eingeleitet wird.

4. Verfahren nach einem der Ansprüche 1 bis 3, **dadurch gekennzeichnet, dass** die Menge eingeleitetes Prozessgas 1 cm³/s bis 100.000 cm³/s, bevorzugt 1 cm³/s bis 10.000 cm³/s, besonders bevorzugt 10 cm³/s bis 1.000 cm³/s beträgt.

5. Verfahren nach einem der Ansprüche 1 bis 4, **dadurch gekennzeichnet, dass** eine getaktete oder kontinuierliche Förderung zumindest einer Teilmenge des mindestens einen Mikroalgenstamms aus der ersten Vorrichtung in Schritt b) in die zweite Vorrichtung in Schritt c) und/oder aus der zweiten Vorrichtung in Schritt c) in die erste Vorrichtung in Schritt b) erfolgt, bevorzugt durch die Einleitung eines Prozessgases in Schritt c) und/oder durch eine Pumpe.

6. Verfahren nach einem der Ansprüche 1 bis 5, **dadurch gekennzeichnet, dass** das Verfahren mindestens einen weiteren Schritt umfasst, wobei der weitere Schritt ausgewählt ist aus einer phototrophen Kultivierung, einer mixotrophen Kultivierung, einer Ernte der Biomasse und/oder dem Trocknen der geernteten Biomasse.

7. System zur simultanen heterotrophen und mixotrophen Kultivierung von Mikroalgen umfassend
i. eine erste Vorrichtung (1), wobei die erste Vorrichtung zur heterotrophen Kultivierung ausgebildet ist,
ii. eine zweite Vorrichtung (2) umfassend ein Rohr aus transluzentem Material und mindestens eine Lichtquelle, wobei die zweite Vorrichtung zur mixotrophen Kultivierung ausgebildet ist,
iii. ein erstes Verbindungselement (3) zur Verbindung der ersten Vorrichtung (1) mit der zweiten Vorrichtung (2),
wobei das erste Verbindungselement (3) den Boden der ersten Vorrichtung (1) mit dem Einlass der zweiten Vorrichtung (2) verbindet,
iv. ein zweites Verbindungselement (4) zur Verbindung der ersten Vorrichtung (1) mit der zweiten Vorrichtung (2),
wobei das zweite Verbindungselement (4) den oberen Teil der ersten Vorrichtung (1) mit dem Auslass der zweiten Vorrichtung (2) verbindet,
wobei die zweite Vorrichtung (2) an dem unteren Ende oder das erste Verbindungselement (3) einen Injektor zur Einleitung eines Prozessgases umfasst.

8. System nach Anspruch 7, **dadurch gekennzeichnet, dass** das aus transluzentem Material bestehende Rohr spiralförmig um ein Trägergestell in Form eines Kegelstumpfs oder in Form eines Hohlzylinders aufsteigend angeordnet ist, wobei das Trägergestell mindestens eine Lichtquelle umfasst.

9. System nach Anspruch 8, **dadurch gekennzeichnet, dass** die zweite Vorrichtung (2) mindestens eine weitere Lichtquelle zur Bestrahlung des Rohrs aus transluzentem Material von außen umfasst, wobei die mindestens eine weitere Lichtquelle bevorzugt ein Flächenstrahler ist.

10. System nach einem der Ansprüche 7 bis 9, **dadurch gekennzeichnet, dass** das transluzente Material ausgewählt ist aus Silikonen, Polyvinylchlorid (PVC), Polyacrylaten, Polyethylen oder Borosilikatglas.

11. System nach Anspruch 10, **dadurch gekennzeichnet, dass** das Rohr aus transluzentem Material aus mindestens einem weiteren Material, ausgewählt aus UVB- und UVC-durchlässigen Materialien besteht.

12. System nach einem der Ansprüche 7 bis 11, **dadurch gekennzeichnet, dass** die mindestens eine Lichtquelle ausgewählt ist aus einer Natriumdampflampe oder einem Leuchtdioden-Strahler, bevorzugt mit einer Wellenlänge im Bereich von 400 nm bis 520 nm oder von 600 nm bis 720 nm und einer Lichtleistung im Bereich von 1 bis 150 W/m².

13. System nach einem der Ansprüche 7 bis 12, **dadurch gekennzeichnet, dass** die zweite Vorrichtung (2) mindestens eine weitere Lichtquelle umfasst, wobei die mindestens eine weitere Lichtquelle eine Wellenlänge im Bereich von 280 nm bis 320 nm aufweist.

14. System nach einem der Ansprüche 7 bis 13, **dadurch gekennzeichnet, dass** das Volumen der ersten Vorrichtung und das Volumen des Rohrs aus transluzentem Material ein Verhältnis im Bereich von 10:1 bis 1:5 aufweisen.

15. Verwendung eines Systems nach einem der Ansprüche 7 bis 14 zur Kultivierung von Mikroalgen.

## Claims

1. Method for the simultaneous heterotrophic and mixotrophic cultivation of microalgae, comprising the steps of
a) providing an inoculum comprising at least one microalgae strain and inoculating a culture medium with the inoculum,
b) cultivating the at least one microalgae strain in a first device under heterotrophic culture conditions,
c) cultivating the at least one microalgae strain in a second device under mixotrophic culture conditions,
wherein at least a portion of the at least one microalgae strain is conveyed from the first device in step b) to the second device in step c) and/or from the second device in step c) to the first device in step b),
wherein a process gas is introduced at the lower end of the second device, wherein the process gas is introduced so as to be at least partially compressed or at least partially condensed.

2. Method according to claim 1, **characterised in that** the process gas in step c) is air or a carbon dioxide-enriched air mixture, preferably an exhaust gas from a heterotrophic cultivation from the first device or produced from technical gases.

3. Method according to claim 1 or 2, **characterised in that** the process gas is introduced in a continuous or clocked manner.

4. Method according to any of claims 1 to 3, **characterised in that** the amount of process gas introduced is 1 cm³/s to 100,000 cm³/s, preferably 1 cm³/s to 10,000 cm³/s, particularly preferably 10 cm³/s to 1,000 cm³/s.

5. Method according to any of claims 1 to 4, **characterised in that** at least a portion of the at least one microalgae strain is conveyed in a continuous or clocked manner from the first device in step b) to the second device in step c) and/or from the second device in step c) to the first device in step b), preferably by introducing a process gas in step c) and/or by means of a pump.

6. Method according to any of claims 1 to 5, **characterised in that** the method comprises at least one further step, the further step being selected from phototrophic cultivation, mixotrophic cultivation, harvesting the biomass and/or drying the harvested biomass.

7. System for the simultaneous heterotrophic and mixotrophic cultivation of microalgae, comprising
i. a first device (1), the first device being designed for heterotrophic cultivation,
ii. a second device (2) comprising a tube made of translucent material and at least one light source, the second device being designed for mixotrophic cultivation,
iii. a first connecting element (3) for connecting the first device (1) to the second device (2),
wherein the first connecting element (3) connects the bottom of the first device (1) to the inlet of the second device (2),
iv. a second connecting element (4) for connecting the first device (1) to the second device (2),
wherein the second connecting element (4) connects the upper part of the first device (1) to the outlet of the second device (2),
wherein the second device (2) comprises an injector for introducing a process gas at the lower end or the first connecting element (3).

8. System according to claim 7, **characterised in that** the tube consisting of translucent material is arranged so as to ascend in a spiral shape around a support frame in the form of a truncated cone or a hollow cylinder, the support frame comprising at least one light source.

9. System according to claim 8, **characterised in that** the second device (2) comprises at least one further light source for irradiating the tube made of translucent material from the outside, the at least one further light source preferably being a surface spotlight.

10. System according to any of claims 7 to 9, **characterised in that** the translucent material is selected from silicones, polyvinyl chloride (PVC), polyacrylates, polyethylene or borosilicate glass.

11. System according to claim 10, **characterised in that** the tube made of translucent material consists of at least one further material selected from UVB- and UVC-permeable materials.

12. System according to any of claims 7 to 11, **characterised in that** the at least one light source is selected from a sodium vapour lamp or an LED spotlight, preferably having a wavelength in the range of from 400 nm to 520 nm or from 600 nm to 720 nm and a light output in the range of 1 to 150 W/m².

13. System according to any of claims 7 to 12, **characterised in that** the second device (2) comprises at least one further light source, the at least one further light source having a wavelength in the range of from 280 nm to 320 nm.

14. System according to any of claims 7 to 13, **characterised in that** the volume of the first device and the volume of the tube made of translucent material have a ratio in the range of from 10:1 to 1:5.

15. Use of a system according to any of claims 7 to 14 for the cultivation of microalgae.

## Revendications

1. Procédé de culture hétérotrophe et mixotrophe simultanée de microalgues, comprenant les étapes de
a) fourniture d'un inoculum comprenant au moins une souche de microalgues et inoculation d'un milieu de culture avec l'inoculum,
b) culture de l'au moins une souche de microalgues dans un premier dispositif dans des conditions de culture hétérotrophes,
c) culture de l'au moins une souche de microalgues dans un second dispositif dans des conditions de culture mixotrophes,
un transport d'au moins une quantité partielle de l'au moins une souche de microalgues étant effectué du premier dispositif à l'étape b) vers le second dispositif à l'étape c) et/ou du second dispositif à l'étape c) vers le premier dispositif à l'étape b),
un gaz de processus étant introduit au niveau de l'extrémité inférieure du second dispositif,
le gaz de processus étant introduit dans un état au moins partiellement comprimé ou au moins partiellement condensé.

2. Procédé selon la revendication 1, **caractérisé en ce que** le gaz de processus à l'étape c) est de l'air ou un mélange d'air enrichi en dioxyde de carbone, de préférence un gaz d'échappement provenant d'une culture hétérotrophe provenant du premier dispositif ou produit à partir de gaz techniques.

3. Procédé selon la revendication 1 ou 2, **caractérisé en ce que** le gaz de processus est introduit de manière cadencée ou continue.

4. Procédé selon l'une des revendications 1 à 3, **caractérisé en ce que** la quantité de gaz de processus introduit est de 1 cm³/s à 100 000 cm³/s, de préférence de 1 cm³/s à 10 000 cm³/s, de manière particulièrement préférée de 10 cm³/s à 1 000 cm³/s.

5. Procédé selon l'une des revendications 1 à 4, **caractérisé en ce qu'**un transport de manière cadencée ou continue d'au moins une quantité partielle de l'au moins une souche de microalgues est effectué du premier dispositif à l'étape b) vers le second dispositif à l'étape c) et/ou du second dispositif à l'étape c) vers le premier dispositif à l'étape b), de préférence par l'introduction d'un gaz de processus à l'étape c) et/ou par une pompe.

6. Procédé selon l'une des revendications 1 à 5, **caractérisé en ce que** le procédé comprend au moins une étape supplémentaire, l'étape supplémentaire étant choisie parmi une culture phototrophe, une culture mixotrophe, une récolte de la biomasse et/ou le séchage de la biomasse récoltée.

7. Système de culture hétérotrophe et mixotrophe simultanée de microalgues, comprenant
i. un premier dispositif (1), le premier dispositif étant conçu pour la culture hétérotrophe,
ii. un second dispositif (2) comprenant un tube en un matériau translucide et au moins une source lumineuse, le second dispositif étant conçu pour la culture mixotrophe,
iii. un premier élément de liaison (3) permettant de relier le premier dispositif (1) au second dispositif (2),
le premier élément de liaison (3) reliant le fond du premier dispositif (1) à l'entrée du second dispositif (2),
iv. un second élément de liaison (4) permettant de relier le premier dispositif (1) au second dispositif (2),
le second élément de liaison (4) reliant la partie supérieure du premier dispositif (1) à la sortie du second dispositif (2),
le second dispositif (2), au niveau de l'extrémité inférieure, ou le premier élément de liaison (3) comprenant un injecteur permettant d'introduire un gaz de processus.

8. Système selon la revendication 7, **caractérisé en ce que** le tube constitué d'un matériau translucide est disposé sous forme de spirale ascendante autour d'une structure de support en forme de cône tronqué ou en forme de cylindre creux, la structure de support comprenant au moins une source lumineuse.

9. Système selon la revendication 8, **caractérisé en ce que** le second dispositif (2) comprend au moins une autre source lumineuse permettant d'irradier le tube en un matériau translucide depuis l'extérieur, l'au moins une autre source lumineuse étant de préférence un projecteur de surface.

10. Système selon l'une des revendications 7 à 9, **caractérisé en ce que** le matériau translucide est choisi parmi les silicones, le polychlorure de vinyle (PVC), les polyacrylates, le polyéthylène ou le verre borosilicate.

11. Système selon la revendication 10, **caractérisé en ce que** le tube en un matériau translucide est constitué d'au moins un autre matériau choisi parmi les matériaux transparents aux rayonnements UVB et UVC.

12. Système selon l'une des revendications 7 à 11, **caractérisé en ce que** l'au moins une source lumineuse est choisie parmi une lampe à vapeur de sodium ou un projecteur à diodes électroluminescentes, de préférence comportant une longueur d'onde dans la plage de 400 nm à 520 nm ou de 600 nm à 720 nm et une puissance lumineuse dans la plage de 1 à 150 W/m².

13. Système selon l'une des revendications 7 à 12, **caractérisé en ce que** le second dispositif (2) comprend au moins une autre source lumineuse, l'au moins une autre source lumineuse présentant une longueur d'onde dans la plage de 280 nm à 320 nm.

14. Système selon l'une des revendications 7 à 13, **caractérisé en ce que** le volume du premier dispositif et le volume du tube en un matériau translucide présentent un rapport dans la plage de 10:1 à 1:5.

15. Utilisation d'un système selon l'une des revendications 7 à 14 pour la culture de microalgues.
